# EUROPÄISCHE PATENTSCHRIFT

(11) **EP 2 838 503 B1**
(45) Veröffentlichungstag und Bekanntmachung des Hinweises auf die Patenterteilung: **13.01.2016**
(21) Anmeldenummer: 13714975.3
(22) Anmeldetag: 11.04.2013
(51) Int. Cl.: A61K 8/89, C08G 77/50, C08L 83/04, C08L 83/07

(54) **ORGANOPOLYSILOXANGELE**
ORGANOPOLYSILOXANE GELS
GELS D'ORGANOPOLYSILOXANE

(30) Priorität: 16.04.2012 DE 102012206209
(43) Veröffentlichungstag der Anmeldung: 25.02.2015
(73) Patentinhaber: Wacker Chemie AG, 81737 München (DE)
(72) Erfinder: KNÖR, Sebastian, 84547 Emmerting (DE); ROY, Aroop Kumar, Mechanicville, NY 12118 (US)
(74) Vertreter: Deffner-Lehner, Maria
(86) Internationale Anmeldenummer: PCT/EP2013/057602
(87) Internationale Veröffentlichungsnummer: WO 2013/156390

(56) Entgegenhaltungen:
- WO-A1-2011/059106
- US-B1- 6 365 670

## Beschreibung

Die Erfindung betrifft Organopolysiloxangele, Verfahren zu deren Herstellung, sowie deren Anwendung in kosmetischen Formulierungen.

Die Organopolysiloxangele der vorliegenden Erfindung werden durch Vernetzung eines ungesättigten Organopolysiloxanharzes mit einem speziellen Si-H-haltigen Organopolysiloxan, im Weiteren auch Si-H-funktioneller Vernetzer genannt, in Gegenwart eines Verdünnungsmittels hergestellt.

Vernetzungen sind Verbindungen von Polymerketten in einem dreidimensionalen Netzwerk. Sie können als langkettige Verzweigungen betrachtet werden, die so zahlreich sind, dass ein kontinuierliches unlösliches Netzwerk oder Gel gebildet wird.

Organopolysiloxannetzwerke werden häufig über platinkatalysierte Hydrosilylierungsreaktionen hergestellt. Dabei werden häufig ein Si-H-haltiges Organopolysiloxan und ein Vinyl-funktionelles Organopolysiloxan miteinander zur Reaktion gebracht. Eine wesentliche Voraussetzung für den Aufbau eines 3-dimensionalen Netzwerkes ist dabei, dass mindestens eine der beiden Komponenten, das Si-H-haltige Organopolysiloxan oder das Vinyl-funktionelles Organopolysiloxan, in der mittleren Zusammensetzung mehr als zwei Funktionalitäten pro Molekül aufweist.

Die platinkatalysierte Hydrosilylierungsreaktion bietet bei der Ausbildung von Organopolysiloxannetzwerken den Vorteil, dass keine Nebenprodukte gebildet werden, und dass Verknüpfungsstellen und Netzwerkarchitektur eng definiert sind.

Der wichtigste Grund für die Anwendung von Organopolysiloxangelen in kosmetischen Anwendungen sind die dadurch erzielten sensorischen Vorteile, insbesondere die Verbesserung des Hautgefühls von kosmetischen Formulierungen. Darüber hinaus dienen Organopolysiloxangele als Verdickungsmittel in kosmetischen Formulierungen.

US 6,423,322 B1 offenbart Organopolysiloxangele, die durch Hydrosilylierungsreaktion eines speziellen, vinylfunktionellen MQ-Harzes mit einem hoch Si-H-haltigen Organopolysiloxan mit ungefähr 0,5 Gew.% siliciumgebundenen Wasserstoffatomen in Gegenwart von Decamethylcyclopentasiloxan als Verdünnungsmittel und einer kleinen Menge Platin-Hydrosilylierungskatalysator leicht hergestellt werden können. Die resultierenden Gele ziehen keine Fäden und lassen sich leicht zu einer stabilen Creme oder Paste homogenisieren. Ein wesentlicher Nachteil dieser Organopolysiloxangele ist jedoch insbesondere, dass das erzeugte Hautgefühl für kosmetische Anwendungen nicht ideal ist.

Darüber hinaus zeigt sich, dass mit vielen organischen Verdünnungsmitteln, die in kosmetischen Anwendungen häufig verwendet werden, keine geeigneten Gele hergestellt werden können, wenn ein Si-H-haltiges Organopolysiloxan mit vergleichsweise hohem Gehalt an siliciumgebundenen Wasserstoffatomen als Si-H-funktioneller Vernetzer verwendet wird. Die teilweise geringe Verträglichkeit der Gele mit organischen Verbindungen und Verdünnungsmitteln ist daher bei der Herstellung kosmetischer Formulierungen nachteilig. Aufgrund des relativ hohen Anteils an vinylfunktionellen MQ-Harz sind solche Gele auch vergleichsweise teuer herzustellen.

US 2004/0105828 A1 und US 2003/0095935 A1 beschreiben eine Reihe von unterschiedlichen Silicongelen, darunter Gele, welche durch Hydrosilylierungsreaktion eines Si-H-haltigen Polysiloxans und einem ungesättigten MQ-Harz in einem Verdünnungsmittel erhalten werden. Als wesentlicher Vorteil wird verbesserte Transfer-Resistenz genannt. Es gibt jedoch keine Beispiele, in den die Herstellung und die Wirkung dieser Organopolysiloxangele gezeigt werden.

Es bestand die Aufgabe, neue Organopolysiloxangele mit verbesserten Eigenschaften, insbesondere mit einem verbesserten Hautgefühl, bereitzustellen, welche die oben genannten Nachteile nicht aufweisen.

Gegenstand der Erfindung sind Organopolysiloxangele hergestellt durch Umsetzung von
(1) ungesättigten MQ-Harze aus Einheiten der Formeln
   SiO₂ (Q-Einheiten) und
   R₃SiO_{1/2} und R₂R'SiO_{1/2} (M-Einheiten),
   wobei R gleich oder verschieden sein kann und einen einwertigen, gegebenenfalls substituierten Kohlenwasserstoffrest mit 1 bis 18 Kohlenstoffatomen je Rest bedeutet,
   R' einen einwertigen Kohlenwasserstoffrest bedeutet, an den Si-H-Gruppen in einer Hydrosilylierungsreaktion angelagert werden können, vorzugsweise ein ω-Alkenylrest mit 2 bis 12 C-Atomen, bevorzugt ein Vinylrest ist,
   mit der Maßgabe, dass die MQ-Harze mindestens 2 Reste R', vorzugsweise mindestens 3 Reste R', enthalten und dass das molare Verhältnis von M-Einheiten zu Q-Einheiten im Bereich von 0,5 bis 4,0, vorzugsweise im Bereich von 0,5 bis 2,0, bevorzugt im Bereich von 0,6 bis 1,5, liegt,
   mit
(2) Si-H funktionellen Organopolysiloxanen der allgemeinen Formel

   H_{c}R_{3-c}SiO(R₂SiO)ₐ(RHSiO)_{b}SiR_{3-c}H_{c} (I),

   wobei
   c 0 oder 1, vorzugsweise 0, ist,
   R gleich oder verschieden sein kann und einen einwertigen, gegebenenfalls substituierten Kohlenwasserstoffrest mit 1 bis 18 Kohlenstoffatomen je Rest bedeutet,
   a und b ganze Zahlen sind, mit der Maßgabe, dass die Summe a+b 66 bis 248, vorzugsweise 98 bis 248, bevorzugt 118 bis 168 ist,
   dass die Organopolysiloxane (2) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten,
   und dass die Anzahl an Si-H-Gruppen pro Molekül in der durchschnittlichen Zusammensetzung größer 2 ist,
   oder Mischungen von (2) Si-H funktionellen Organopolysiloxanen mit
(2') Si-H funktionellen Organopolysiloxanen der allgemeinen Formel

   H_{c}R_{3-c}SiO(R₂SiO)ₐ(RHSiO)_{b}SiR_{3-c}H_{c} (I'),

   wobei
   c 0 oder 1, vorzugsweise 0 ist,
   R die oben dafür angegebene Bedeutung hat,
   a und b ganze Zahlen sind, mit der Maßgabe, dass die Summe a+b 8 bis 248, vorzugsweise 38 bis 248, ist, und dass die Organopolysiloxane (2') Si-gebundenen Wasserstoff in Mengen von 0,045 bis 0,35 Gew.-%, vorzugsweise von 0,045 bis 0,156 Gew.-%, enthalten,
   mit der Maßgabe, dass wenn Mischungen von (2) und (2') eingesetzt werden, das Gewichts-Verhältnis von (2) zu (2') vorzugsweise größer 0,2, besonders bevorzugt größer 0,3 ist,
   in Gegenwart von
(3) die Anlagerung von Si-gebundenem Wasserstoff an aliphatische Mehrfachbindung fördernde Katalysatoren,
   wobei (1) und (2) oder Mischungen von (2) und (2') in
(4) Verdünnungsmitteln ausgewählt aus Organopolysiloxanen mit 2 bis 200 Si-Atomen, bevorzugt Organopolysiloxanen mit 2 bis 50 Si-Atomen, und organischen Verdünnungsmitteln, dispergiert sind.

Gegenstand der Erfindung ist weiterhin ein Verfahren zur Herstellung der erfindungsgemäßen Organopolysiloxangele indem
(1) ungesättigte MQ-Harze aus Einheiten der Formeln
   SiO₂ (Q-Einheiten) und
   R₃SiO_{1/2} und R₂R'SiO_{1/2} (M-Einheiten),
   wobei R gleich oder verschieden sein kann und einen einwertigen, gegebenenfalls substituierten Kohlenwasserstoffrest mit 1 bis 18 Kohlenstoffatomen je Rest bedeutet,
   R' einen einwertigen Kohlenwasserstoffrest bedeutet, an den Si-H-Gruppen in einer Hydrosilylierungsreaktion angelagert werden können, vorzugsweise ein ω-Alkenylrest mit 2 bis 12 C-Atomen, bevorzugt ein Vinylrest ist,
   mit der Maßgabe, dass die MQ-Harze mindestens 2 Reste R', vorzugsweise mindestens 3 Reste R', enthalten und dass das molare Verhältnis von M-Einheiten zu Q-Einheiten im Bereich von 0,5 bis 4,0, vorzugsweise im Bereich von 0,5 bis 2,0,
   bevorzugt im Bereich von 0,6 bis 1,5, liegt,
   mit
(2) Si-H funktionellen Organopolysiloxanen der allgemeinen Formel

   H_{c}R_{3-c}SiO(R₂SiO)ₐ(RHSiO)_{b}SiR_{3-c}H_{c} (I),

   wobei
   c 0 oder 1, vorzugsweise 0, ist,
   R gleich oder verschieden sein kann und einen einwertigen, gegebenenfalls substituierten Kohlenwasserstoffrest mit 1 bis 18 Kohlenstoffatomen je Rest bedeutet,
   a und b ganze Zahlen sind, mit der Maßgabe, dass die Summe a+b 66 bis 248, vorzugsweise 98 bis 248, bevorzugt 118 bis 168 ist,
   dass die Organopolysiloxane (2) Si-gebundenen Wasserstoff in Mengen von 0,011 bis 0,044 Gew.-%, vorzugsweise von
   0,019 bis 0,044 Gew.-%, bevorzugt von 0,022 bis 0,032 Gew.-%, enthalten,
   und dass die Anzahl an Si-H-Gruppen pro Molekül in der durchschnittlichen Zusammensetzung größer 2 ist,
   oder Mischungen von (2) Si-H funktionellen Organopolysiloxanen mit
(2') Si-H funktionellen Organopolysiloxanen der allgemeinen Formel

   H_{c}R_{3-c}SiO(R₂SiO)ₐ(RHSiO)_{b}SiR_{3-c}H_{c} (I'),

   wobei
   c 0 oder 1, vorzugsweise 0 ist,
   R die oben dafür angegebene Bedeutung hat,
   a und b ganze Zahlen sind, mit der Maßgabe, dass die Summe a+b 8 bis 248, vorzugsweise 38 bis 248, ist, und dass die Organopolysiloxane (2') Si-gebundenen Wasserstoff in Mengen von 0,045 bis 0,35 Gew.-%, vorzugsweise von 0,045 bis 0,156 Gew.-%, enthalten,
   mit der Maßgabe, dass wenn Mischungen von (2) und (2') eingesetzt werden, das Gewichts-Verhältnis von (2) zu (2') vorzugsweise größer 0,2, besonders bevorzugt größer 0,3 ist,
   in Gegenwart von
(3) die Anlagerung von Si-gebundenem Wasserstoff an
   aliphatische Mehrfachbindung fördernde Katalysatoren, umgesetzt werden,
   wobei (1) und (2) oder Mischungen von (2) und (2') in
(4) Verdünnungsmitteln ausgewählt aus Organopolysiloxanen mit 2 bis 200 Si-Atomen, bevorzugt Organopolysiloxanen mit 2 bis 50 Si-Atomen, und organischen Verdünnungsmitteln, dispergiert sind.

Im Rahmen dieser Erfindung sollen die Formeln (I) und (I') so verstanden werden, dass a Einheiten -(R₂SiO)- und b Einheiten - (RHSiO)- in beliebiger Weise im Organopolysiloxanmolekül verteilt sein können.

Die erfindungsgemäß eingesetzten Si-H-haltigen Organopolysiloxane (2) haben vorzugsweise eine Viskosität von 50 bis 2000 mm²/s, bevorzugt 100 bis 1000 mm²/s, besonders bevorzugt 150 bis 600 mm²/s, jeweils bei 25°C, und ein molares Verhältnis a:(b+c) von vorzugsweise 30:1 bis 150:1, bevorzugt 30:1 bis 80:1, besonders bevorzugt 40:1 bis 70:1. Die in den Mischungen mit (2) eingesetzten Si-H-haltigen Organopolysiloxane (2') haben vorzugsweise eine Viskosität von 3 bis 2000 mm²/s, besonders bevorzugt 20 bis 1200 mm²/s, jeweils bei 25°C, und ein molares Verhältnis a:(b+c) von vorzugsweise 4:1 bis 30:1, besonders bevorzugt 8:1 bis 30:1.

Überraschenderweise wurde gefunden, dass die erfindungsgemäßen Organopolysiloxangele auf Basis der Si-H-haltigen Organopolysiloxane (2) oder auf Basis der Mischung von (2) und (2') wesentlich bessere sensorische Eigenschaften, insbesondere ein besseres Hautgefühl aufweisen, als Gele auf Basis eines Organopolysiloxans mit relativ hohen Gehalt an siliciumgebundenen Wasserstoffatomen, wie sie in US 6,423,322 B1 offenbart sind. Sie sind außergewöhnlich gleitfähig und fühlen sich nicht unerwünscht ölig an. Nach dem Verteilen auf der Haut hinterlassen sie ein geschmeidigeres Hautgefühl und lassen kein unerwünscht filmartiges oder stumpfes Gefühl zurück.

Beispiele für Reste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der α- und der β-Phenylethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest.

Bevorzugt handelt es sich bei dem Rest R um einen einwertigen Kohlenwasserstoffrest mit 1 bis 6 Kohlenstoffatomen, wobei der Methylrest besonders bevorzugt ist.

Die ungesättigten MQ-Harze (1) können bis zu 10 Gew.-% freie Hydroxy- oder Alkoxygruppen enthalten.

Vorzugsweise haben die ungesättigten MQ-Harze (1) bei 25 °C eine Viskosität größer als 0,7 mm²/s, besonders bevorzugt sind solche Harze, die bei 25 °C eine Viskosität von größer als 1000 mm²/s haben oder Feststoffe sind. Das mit Gelpermeationschromatografie bestimmte gewichtsmittlere Molekulargewicht M_{w} (bezogen auf einen Polystyrolstandard) dieser Harze beträgt vorzugsweise 334 bis 200000 g/mol, bevorzugt 1000 bis 20000 g/mol.

Die ungesättigten MQ-Harze (1) der erfindungsgemäßen Organopolysiloxangele haben vorzugsweise eine Jodzahl kleiner 254 und bevorzugt sind Organopolysiloxanharze mit einer Jodzahl kleiner 76. Der ungesättigte Kohlenwasserstoffrest ist vorzugsweise gebunden an eine M-Einheit (=M_{Vi}) oder D-Einheit (=D_{Vi}), bevorzugt an eine M-Einheit, wobei das molare Verhältnis M:(M_{Vi}+D_{Vi}), vorzugsweise M:M_{Vi}, vorzugsweise im Bereich 0 bis 50, bevorzugt im Bereich 0 bis 20, besonders bevorzugt im Bereich 2,5 bis 13 liegt.

Beispiele für Reste R' sind Alkenylreste, wie der Vinyl-, 5-Hexenyl-, Cyclohexenyl-, 1-Propenyl-, Allyl-, 3-Butenyl- und 4-Pentenylrest, und Alkinylreste, wie der Ethinyl-, Propargyl- und 1-Propinylrest. Bevorzugt handelt es sich bei dem Rest R' um Alkenylreste, besonders bevorzugt ω-Alkenylreste, insbesondere um den Vinylrest.

Bei den erfindungsgemäßen Organopolysiloxangelen werden ungesättigte MQ-Harze (1) in Mengen von vorzugsweise 4,5 bis 0,1 Mol, bevorzugt 2 bis 0,8 Mol, besonders bevorzugt 1,8 bis 1,1 Mol, Kohlenwasserstoffrest mit aliphatischer C-C-Mehrfachbindung je Mol Si-gebundenem Wasserstoff in Si-H funktionellen Organopolysiloxanen (2) und (2') eingesetzt.

Das gewichtsmäßige Verhältnis von MQ Harz zum Si-H-haltigen Organopolysiloxan in den in US 6,423,322 B1 offenbarten Organopolysiloxangelen liegt im Bereich von 7 bis 4. Der hohe Anteil an vergleichsweise teurem Harz macht diese Gele vergleichsweise teuer. In den erfindungsgemäßen Organopolysiloxangelen liegt das gewichtsmäßige Verhältnis von ungesättigten MQ-Harzen (1) zu Si-H-haltigen Organopolysiloxanen (2) vorzugsweise im Bereich von 3 bis 0,1, bevorzugt im Bereich 2,0 bis 0,1, besonders bevorzugt im Bereich 1 bis 0,1. In den erfindungsgemäßen Organopolysiloxangelen liegt das gewichtsmäßige Verhältnis von ungesättigten MQ-Harzen (1) zu den Mischungen der Si-H-haltigen Organopolysiloxane (2) und (2') vorzugsweise im Bereich von 3 bis 0,1, bevorzugt im Bereich 2,5 bis 0,1, besonders bevorzugt im Bereich 2,2 bis 0,1.

Als Katalysator (3) können bei dem erfindungsgemäßen Verfahren die gleichen Katalysatoren eingesetzt werden, die auch bisher zur Förderung der Anlagerung von Si-gebundenem Wasserstoff an aliphatische Mehrfachbindung eingesetzt werden konnten. Bei den Katalysatoren handelt es sich vorzugsweise um ein Metall aus der Gruppe der Platinmetalle oder um eine Verbindung oder einen Komplex aus der Gruppe der Platinmetalle. Beispiele für solche Katalysatoren sind metallisches und feinverteiltes Platin, das sich auf Trägern wie Siliciumdioxid, Aluminiumoxid oder Aktivkohle befinden kann, Verbindungen oder Komplexe von Platin, wie Platinhalogenide, z. B. PtCl₄, H₂PtCl₆•6H₂O, Na₂PtCl₄•4H₂0, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus H₂PtCl₆•6H₂0 und Cyclohexanon, Platin-Vinyl-siloxankomplexe, wie Platin-1,3-Divinyl-1,1,3,3-tetra-methyl-disiloxankomplexe mit oder ohne Gehalt an nachweisbarem anorganisch gebundenen Halogen, Bis-(y-picolin)-platindichlorid, Trimethylendipyridinplatindichlorid, Dicyclopentadienplatindichlorid, Dimethylsulfoxidethylenplatin-(II)-dichlorid, Cyclooctadien-Platindichlorid, Norbornadien-Platindichlorid, γ-picolin-Platindichlorid, Cyclopentadien-Platindichlorid, sowie Umsetzungsprodukte von Platintetrachlorid mit Olefin und primärem Amin oder sekundärem Amin oder primärem und sekundärem Amin, wie das Umsetzungsprodukt aus in 1-0cten gelöstem Platintetrachlorid mit sec.-Butylamin oder Ammonium-Platinkomplexe. Bevorzugte Hydrosilylierungskatalysatoren sind Platinverbindungen, die in einem zur Verwendung in kosmetischen Formulierungen geeigneten Lösungsmittel vorliegen.

Bevorzugt wird der Katalysator (3) in Mengen 1 bis 50 Gew.-ppm (Gewichtsteilen je Million Gewichtsteilen), besonders bevorzugt 2 bis 20 Gew.-ppm, jeweils berechnet als elementares Platin und bezogen auf das Gesamtgewicht der ungesättigten MQ-Harze (1), der Si-H funktionellen Organopolysiloxane (2) oder der Mischung der Si-H funktionellen Organopolysiloxane (2) und (2') und dem Verdünnungsmittel (4).

Die erfindungsgemäßen Organopolysiloxangele enthalten vorzugsweise 1 bis 98 Gew.-% Verdünnungsmittel, bevorzugt 50 bis 95 Gew.-% Verdünnungsmittel, bezogen auf das Gesamtgewicht der Organopolysiloxangele.

Nicht-reaktive oder relativ nicht-reaktive Verdünnungsmittel sind bevorzugt. Im Rahmen der vorliegenden Erfindung wird der Begriff "nicht-reaktiv" in Bezug auf die in Frage stehende Vernetzungsreaktion und die hierin eingesetzten Reaktanden verwendet. Ein relativ nichtreaktives Verdünnungsmittel ist weniger als ein Zehntel so reaktiv mit den Reaktanden der Vernetzungsreaktion im Vergleich zu den Reaktanden untereinander in der Vernetzungsreaktion.

Geeignete Beispiele von Verdünnungsmittel beinhalten cyclische und lineare Organopolysiloxane, organische Verdünnungsmittel oder Mischungen von Organopolysiloxanen und organischen Verdünnungsmitteln.

Das Organopolysiloxan kann ein einzelnes Organopolysiloxan oder eine Mischung von Organopolysiloxanen sein. Das Organopolysiloxan kann Alkyl-, Aryl-, Alkaryl- und Aralkylgruppen tragen. Solche Organopolysiloxane können beispielhaft durch Polydimethylsiloxan, Polydiethylsiloxan, Polymethylethylsiloxan, Polymethylphenylsiloxan und Polydiphenylsiloxan angegeben werden, sind aber nicht darauf beschränkt.

Möglich ist auch die Verwendung von funktionellen Organopolysiloxanen, beispielsweise acrylamidfunktionelle Siloxanfluide, acrylfunktionelle Siloxanfluide, amidfunktionelle Siloxanfluide, aminofunktionelle Siloxanfluide, carbinolfunktionelle Siloxanfluide, carboxyfunktionelle Siloxanfluide, chloralkylfunktionelle Siloxanfluide, epoxfunktionelle Siloxanfluide, glykolfunktionelle Siloxanfluide, ketalfunktionelle Siloxanfluide, mercaptofunktionelle Siloxanfluide, methylesterfunktionelle Siloxanfluide, perfluorofunktionelle Siloxanfluide und silanofunktionelle Siloxane.

Cyclische Polydimethylsiloxane können beispielhaft durch Hexamethylcyclotrisiloxan, Octamethylcyclotetrasiloxan, Decamethylcyclopentasiloxan und Dodecamethylcyclohexasiloxan angegeben werden, sind aber nicht darauf beschränkt. Vorzugsweise ist das Organopolysiloxan ein Polydimethylsiloxan mit 2 bis 200 Si-Atomen, bevorzugt 2 bis 50 Si-Atomen, besonders bevorzugt sind lineare Polydimethylsiloxane mit einer Viskosität von 1,5 bis 50 mm²/s bei 25°C.

Als organische Verdünnungsmittel können aromatische Kohlenwasserstoffe, Alkohole, Aldehyde, Ketone, Amine, Ester, Ether, Alkylhalogenide oder aromatische Halogenide verwendet werden. Stellvertretende Beispiele sind Alkohole, wie Methanol, Ethanol, i-Propanol, Cyclohexanol, Benzylalkohol, 2-Octanol, Ethylenglykol, Propylenglykol und Glycerin; aliphatische Kohlenwasserstoffe, wie Pentan, Cyclohexan, Heptan, Lackbenzine; Alkylhalogenide wie Chloroform, Kohlenstofftetrachlorid, Perchlorethylen, Ethylchlorid und Chlorbenzol; aromatische Kohlenwasserstoffe, wie Benzol, Toluol, Ethylbenzol und Xylol; Ester von Carbonsäuren mit 2 bis 30 C-Atomen, wie Ethylacetat, Isopropylacetat, Ethylacetoacetat, Amylacetat, Isobutylisobutyrat, Benzylacetat, Isopropylpalmitat und Isopropylmyristat (=Myristinsäureisopropylester); Ether, wie Ethylether, n-Butylether, Tetrahydrofuran und 1,4-Dioxan; Ketone, wie Aceton, Methylethylketon, Cyclohexanon, Diacetonalkohol, Methylamylketon und Diisobutylketon; Fettöle einschließlich mehrfach ungesättigter ω-3- und ω-6-Fettsäuren, und deren Ester; pflanzliche Öle, wie Erdnuss-, Oliven-, Palm-, Canola-, Maiskeim-, Soja-, Sonnenblumenöl und dergleichen; und natürliche und synthetische Öle oder öllösliche Feststoffe, wie verschiedene Mono-, Di- und Triglyceride, polyoxyalkylierte pflanzliche Öle, Lanolin, Lecithin und dergleichen; und Erdölkohlenwasserstoffe, wie Petrolatum, Mineralöl, Benzin, Petrolether. Diese Beispiele dienen der Erläuterung und sind nicht als Einschränkung zu verstehen.

Andere gemischte organische Verdünnungsmittel können auch verwendet werden, wie Acetonitril, Nitromethan, Dimethylformamid, Propylenoxid, Trioctylphosphat, Butyrolacton, Furfural, Pinienöl, Terpentin und m-Cresol.

Geeignete organische Verdünnungsmittel sind auch flüchtige Aromastoffe, wie Pfefferminzöl, Öl von grüner Minze, Menthol, Vanille, Zimtöl, Nelkenöl, Lorbeeröl, Anisöl, Eukalyptusöl, Thymianöl, Zedernöl, Öl von der Muskatnuss, Öl von Salbei, Kassiaöl, Kakao, Süßholzsaft, Stärkezuckersirup aus Mais mit hohem Fructosegehalt, Citrusöle, wie Zitrone, Orange, Limone und Grapefruit, Fruchtessenzen, wie Apfel, Birne, Pfirsich, Traube, Erdbeere, Himbeere, Kirsche, Pflaume, Ananas und Aprikose; und andere nützliche Aromastoffe einschließlich Aldehyde und Ester, wie Zimtsäureessigester, Zimtaldehyd, Eugenylformat, p-Methylanisol, Acetaldehyd, Benzaldehyd, Anisaldehyd, Citral, Neral, Decanal, Vanillin, Tolylaldehyd, 2,6-Dimethyloctanal und 2-Ethylbutyraldehyd.

Ein Teil oder das gesamte organische Verdünnungsmittel kann ein oder mehrere flüchtige Duftstoffe umfassen, wie natürliche Produkte und Parfumöle. Einige stellvertretende natürliche Produkte und Parfumöle sind Amber, Benzoin, Zibet, Nelke, Zedernöl, Jasmin, Mate, Mimose, Moschus, Myrrhe, Iris, Sandelholzöl und Vetiveröl; Aromachemikalien, wie Amylsalicylat, Amylzimtaldehyd, Benzylacetat, Citronellol, Cumarin, Geraniol, Isobornylacetat, Ambrette und Terpinylacetat, und verschiedene klassische Parfümölfamilien, wie die Blumenbouquetfamilie, die orientalische Familie, die Chyprefamilie, die Holzfamilie, die Citrusfamilie, die Canoefamilie, die Lederfamilie, die Gewürzfamilie und die Kräuterfamilie.

Das organische Verdünnungsmittel kann auch aliphatische oder alicyclische Kohlenwasserstoffe mit 4 bis 30 C-Atomen, vorzugsweise gesättigte Kohlenwasserstoffe, umfassen. Die aliphatischen Kohlenwasserstoffe können geradkettig oder verzweigt sein, und die alicyclischen Kohlenwasserstoffe können unsubstituierte cyclische Kohlenwasserstoffe oder aliphatische kohlenwasserstoffsubstituierte Kohlenwasserstoffe darstellen. Beispiele für geeignete Kohlenwasserstoffe sind n-Heptan, n-Octan, Isooctan, n-Decan, Isodecan, n-Dodecan, Isododecan, Cyclohexan, Cycloheptan, Cyclooctan, Methylcyclohexan, Dimethylcyclohexan, Ethylcyclohexan, Nonylcyclohexan und dergleichen. Auch diese Aufzahlung dient der Erläuterung und ist nicht als Einschränkung zu verstehen.

Weitere geeignete organische Verdünnungsmittel sind ölartige Polyether wie Bis(alkyl)ether von niedermolekularen Glykolen und flüssige oligomere und polymere Polyoxyalkylenglykole, deren Alkylmono- und -diether und Mono- und Dialkylester. Vorzugsweise wird der überwiegende Teil der Polyoxyalkylenglykole aus einem überwiegenden Teil (> 50 Mol-%) von Alkylenoxiden mit mehr als zwei Kohlenstoffatomen, d. h. Propylenoxid, 1,2- und 2,3-Butylenoxid, Tetrahydrofuran, Oxetan, Cyclohexenoxid und dergleichen, hergestellt.

Bevorzugte organische Verdünnungsmittel weisen eine Viskosität im Bereich von 0,5 bis 200 mm²/s (25°C) auf, wobei solche Verdünnungsmittel mit einen Siedepunkt im Bereich von 50°C bis 300°C besonders bevorzugt sind.

Es können zahlreiche Mischungen von Verdünnungsmittel verwenden werden, die lediglich auf diejenigen Zusammensetzungen beschränkt sind, bei denen nach der Herstellung des erfindungsgemäßen Organopolysiloxangels keine Phasentrennung auftritt.

Völlig überraschend wurde gefunden, dass die erfindungsgemäßen Gele, hergestellt mit einem Si-H-haltigen Organopolysiloxan (2) oder einer Si-H-haltigen Organopolysiloxan-Mischung von (2) und (2'), eine verbesserte Kompatibilität zu organischen Verdünnungsmitteln aufweisen. Es werden cremige und lagerstabile Gele mit hervorragender Eignung für kosmetische Anwendungen erhalten. Dagegen konnten keine geeigneten und lagerstabile Gele in organischen Verdünnungsmittel hergestellt werden, wenn Si-H-haltige Organopolysiloxane mit vergleichsweise hohem Gehalt an siliciumgebundenen Wasserstoff, wie in US 6,423,322 B1 offenbart, verwendet wurden.

Die Herstellung des Gels ist leicht durchführbar. Im Allgemeinen gibt man alle Bestandteile außer dem Katalysator zu, rührt langsam, bis sich eine homogene Mischung ergibt, und setzt dann unter kontinuierlichem Rühren den Katalysator zu. Die Zusammensetzung kann bei Raumtemperatur belassen werden, bis sich ein Gel gebildet hat, oder erhitzt werden. Vorzugsweise erhitzt man die Zusammensetzung auf eine Temperatur zwischen 50°C und 130°C und bevorzugt zwischen 70°C und 120°C, bis die Mischung geliert oder fest wird. Die Gelierung erfolgt vorzugsweise innerhalb von zehn Stunden, bevorzugt innerhalb von drei Stunden. Es werden Organopolysiloxangele, welche für die Anwendung in kosmetischen Formulierungen geeignet sind, erhalten.

In einem optionalen zweiten Verfahrensschritt wird das im ersten Verfahrensschritt erhaltene erfindungsgemäße Organopolysiloxangel unter Verwendung von standardmäßigen hochscherenden Mischtechniken bis zu einer cremigen Konsistenz homogenisiert. Dies kann durch intensives Mischen und Dispergieren in Rotor-Stator-Rührvorrichtungen, Kolloidmühlen, Hochdruckhomogenisatoren, Mikrokanälen, Membranen, Strahldüsen und ähnlichem, oder mittels Ultraschall erfolgen. Homogenisiergeräte und Verfahren sind z.B. in Ullmann's Encyclopedia of Industrial Chemistry, CD-ROM-Ausgabe 2003, Wiley-VCH Verlag, unter dem Stichwort "Emulsions" beschrieben. In einem optionalen dritten Verfahrensschritt wird eine weitere Menge Verdünnungsmittel zu dem nach den ersten oder optionalen zweiten Verfahrensschritten erhaltenen Organopolysiloxangel gegeben. Dadurch ist es möglich, ausgehend von einem im ersten Verfahrensschritt erhaltenen "Basisgel" eine Vielzahl unterschiedlicher Gele herzustellen, die in ihrer Konsistenz und ihrem Eigenschaftsprofil in einem breiten Bereich variieren. Man kann dabei das gleiche Verdünnungsmittel verwenden, welches im ersten Verfahrensschritt verwendet worden ist, oder ein zweites Verdünnungsmittel beinhaltend die vorhergehend hierin als Verdünnungsmittel beschriebenen. Alternativ kann auch eine beliebige Mischung der vorhergehend hierin beschriebenen Verdünnungsmittel und/oder ein aktiver Wirkstoff für die Körperpflege oder Gesundheitspflege oder eine Mischung eines aktiven Wirkstoffs für die Körperpflege oder Gesundheitspflege mit einem oder mehreren der hierin beschriebenen Verdünnungsmitteln zugegeben werden, mit der Maßgabe, dass keine Phasentrennung auftritt.

Ein "aktiver Wirkstoff für die Körperpflege oder Gesundheitspflege" bedeutet im vorliegenden Zusammenhang eine beliebige Verbindung oder Mischung von Verbindungen, die im Fachgebiet als Zusatzstoffe in Körperpflegeformulierungen bekannt sind und die typischerweise zugesetzt werden, um das Haar oder die Haut zu behandeln, um einen kosmetischen und/oder ästhetischen Nutzen zu erzielen, eine beliebige Verbindung oder Mischung von Verbindungen, die im Fachgebiet bekannt sind, um einen pharmazeutischen oder medizinischen Nutzen zu erzielen; eine beliebige Verbindung, mit der eine pharmakologische Wirksamkeit oder einen sonstigen Effekt bei der Diagnose, Heilung, Linderung, Behandlung oder Vorbeugung von Krankheiten erzielt werden soll, oder um die Struktur oder eine beliebige Funktion des Körpers eines Menschen oder eines Tiers zu beeinflussen; und jede beliebige Verbindung, die bei der Herstellung von Arzneimittelprodukten eine chemische Veränderung erfahren kann und die in Arzneimitteln in modifizierter Form vorliegen kann, um die angegebene Wirksamkeit oder den angegebenen Effekt hervorzurufen. So umfasst ein "aktiver Wirkstoff für die Körperpflege oder Gesundheitspflege" einen aktiven Wirkstoff oder aktiven Arzneimittelbestandteil wie allgemein von der United States Department of Health & Human Services Food and Drug Administration, Titel 21, Kapitel I, des Code of Federal Regulations, Teile 200 - 299 sowie Teile 300-499 definiert, ist jedoch nicht hierauf beschränkt.

Die aktiven Wirkstoffe für die Körperpflege oder Gesundheitspflege sind vorzugsweise ausgewählt aus der Gruppe der fett- oder öllöslichen Vitamine, öllöslichen Arzneimittel, wobei Antiaknemittel, antibakterielle Mittel, fungizide Mittel, entzündungshemmende Mittel, schuppenflechtebekämpfende Mittel, Betäubungsmittel, juckreizlindernde Mittel, hautentzündungshemmende Mittel und Mittel, die im Allgemeinen als Sperrfilme betrachtet werden besonders bevorzugt sind, und öllöslichen UV-Absorber.

Nützliche aktive Bestandteile zur Verwendung im Schritt 3 des Verfahrens gemäß der Erfindung umfassen fett- als auch öllösliche Vitamine. Nützliche, öllösliche Vitamine umfassen, sind, aber nicht beschränkt auf, Vitamin A₁, RETINOL, C₂- bis C₁₈-Ester von RETINOL, Vitamin E, TOCOPHEROL, Ester von Vitamin E und Mischungen derselben. RETINOL umfasst trans-RETINOL, 13-cis-RETINOL, ll-cis-RETINOL, 9-cis-RETINOL und 3,4-Didehydro-RETINOl. Das öllösliche Vitamin kann in der Zusammensetzung gemäß der Erfindung in Mengen von 0,01 bis 50 Gewichtsprozent verwendet werden.

Es sollte bemerkt werden, dass RETINOL ein International Nomenclature Cosmetic Ingredient Name (INCI), vergeben von The Cosmetic, Toiletry and Fragrance Association (CTFA), Washington DC, für Vitamin A ist. Andere geeignete Vitamine und die INCI-Namen für die in Betracht stehenden Vitamine, die hierin umfasst sind, sind RETINYL ACETAT, RETINYL PALMITATE, RETINYL PROPIONATE, a- TOCOPHEROL,TOCOPHERSOLAN,TOCOPHERYL ACETATE, TOCOPHERYLLINOLEATE, TOCOPHERYLNICOTINATE und TOCOPHERYL SUCCINATE.

Einige Beispiele für kommerziell erhältliche Produkte, die zur Verwendung hierin geeignet sind, sind Vitamin-A-Acetat, Fluka Chemie AG, Buchs, Schweiz; CIOVI-OX T-50, ein Vitamin E-Produkt von Henkel Corporation, La Grange, Illinois; COVI-OX T-70, ein anderes Vitamin-E-Produkt von Henkel Corporation, La Grange Illinois, und Vitamin-E-Acetat, ein Produkt von Roche Vitamins & Fine Chemicals, Nutley, New Jersey.

Stellvertretende Beispiele für einige geeignete öllösliche Arzneimittel, welche als aktive Bestandteile im dritten Verfahrensschritt gemäß der Erfindung zugefügt werden können, sind Clonidin, Scopolamin, Propranolol, Estradiol, Phenylpropanolaminhydrochlorid, Ouabain, Atropin, Haloperidol, Isosorbid, Nitroglycerin, Ibuprofen, Ubichinone, Indomethacin, Prostaglandine, Naproxen, Salbutamol, Guanabenz, Labetalol, Pheniramin, Metrifonat und Steroide.

Ebenso hierin umfasst als ein Arzneimittel für die Zwecke der vorliegenden Erfindung sind Antiaknemittel wie Benzoylperoxid, Triclosan und Tretinoin; antibakterielle Mittel wie Chlorhexidingluconat; fungizide Mittel wie Miconazolnitrat; entzündungshemmende Mittel wie Salicylsäure; corticosteroide Arzneimittel; nichtsteroide entzündungshemmende Mittel wie Diclofenac; schuppenflechtebekämpfende Mittel wie Clobetasolpropionat und Retinoide, Betäubungsmittel wie Lidocain; juckreizlindernde Mittel wie Polidocanol; hautentzündungshemmende Mittel wie Prednisolon und Mittel, die im Allgemeinen als Sperrfilme betrachtet werden.

Stellvertretende Beispiele für öllösliche UV-Absorber, welche als aktive Bestandteile im dritten Verfahrensschritt gemäß der Erfindung zugefügt werden können, sind 1-(4-Methoxyphenyl)-3-(4-tert-butylphenyl)propan-1,3-dione (INCI: Butyl Methoxydibenzoylmethan), 2-Ethylhexyl-(2E)-3-(4-methoxyphenyl)prop-2-enoat (INCI: Octyl Methoxycinnamat), 4-Hydroxy-2-methoxy-5-(oxo-phenylmethyl)benzenesulfonic acid (INCI: Benzophenon-4), 2-Hydroxy-4-methoxybenzophenon-5-sulfonsäure Natriumsalz (INCI: Benzophenon-5) und 2-Ethylhexyl-2-hydroxybenzoat(INCI: Ethylhexylsalicylat).

Bevorzugt wird in einem vierten Verfahrensschritt das nach dem ersten oder optionalen zweiten oder optionalen dritten Verfahrensschritt erhaltene erfindungsgemäße Organopolysiloxangel unter Verwendung von standardmäßigen hochscherenden Mischtechniken bis zu einer cremigen Konsistenz homogenisiert. Hierfür geeignete Technologien sind oben genannt. Wenn im optionalen dritten Verfahrensschritt eine zusätzliche Menge Verdünnungsmittel zugegeben worden ist, so wird dieses im vierten Verfahrensschritt homogen im Gel verteilt. Das Gel quillt und verändert seine Weichheit.

Unter "cremig" in Bezug auf das Gel ist zu verstehen, dass das Ausgangsgel bis zu einer cremigen Konsistenz geschert worden ist. Das resultierende cremige Gel kann je nachdem gießbar oder verhältnismäßig steif sein. Durch das Attribut "cremig" unterscheiden sich diese gescherten Gele, die transparent oder opak sein können, von den unmittelbar durch Gelierung der reaktiven Bestandteile hergestellten Gelen.

Unter "lagerstabil" im Rahmen dieser Erfindung ist zu verstehen, dass sich die gebildeten Organopolysiloxangele innerhalb von 6 Monaten Lagerung bei Raumtemperatur nicht in zwei oder mehr Phasen entmischen und sich die Weichheit des Gels in diesem Zeitraum nicht wesentlich ändert.

Dem erfindungsgemäßen Organopolysiloxangel wird vorzugsweise ein Hydrosilylierungskatalysatorgift oder ein SiH-Quencher zugesetzt, wodurch die Nachhärtung, die durch verbleibende vernetzende Hydrosilylierungsreaktionen, die in den Siliconelastomeren auftreten, bewirkt wird, beendet wird. Beispielhaft für Hydrosilylierungskatalysatorgifte oder SiH-Quencher, welche geeignet sind um die Nachhärtung zu beenden, sind schwefelorganische Verbindungen. Weitere geeignete Verbindungen sind in US 6,200,581 genannt. Bevorzugte Hydrosilylierungskatalysatorgifte sind Mercaptoalkylorganopolysiloxanen, besonders bevorzugt sind mercaptopropylfunktionelle Silsesquisiloxane oder mercaptopropylfunktionelle Polyorganosiloxane, welche vorzugsweise in Mengen von 200 bis 1,0 Mol, bevorzugt 50 bis 1,5 Mol, besonders bevorzugt 20 bis 2,0 Mol, Mercaptogruppen je Mol Platinatome eingesetzt werden. Die Zugabe des Hydrosilylierungskatalysatorgifts oder des SiH-Quenchers kann beliebig in einem oder mehreren der genannten Verfahrensschritte erfolgen.

Die erfindungsgemäßen Organopolysiloxangele sind besonders bevorzugt geeignet für kosmetische Anwendungen, und werden daher bevorzugt in kosmetischen Zusammensetzungen eingesetzt. Sie eignen sich aber auch für andere Anwendungen, beispielsweise für medizinische und technische Anwendungen.

Die Organopolysiloxangele haben besonderen Wert in Körperpflegeprodukten. Sie können sanft auf der Haut verteilt werden und können daher alleine verwendet werden oder mit anderen Körperpflegeproduktbestandteilen gemischt werden, um eine Vielzahl von Körperpflegeprodukten zu bilden.

Beispiele von Körperpflegeproduktbestandteilen sind Ester, Wachse, Öle und Fette von tierischem oder pflanzlichem Ursprung, Fettalkohole, Fettsäuren, Alkylester von Fettsäuren, Kohlenwasserstoffe und -wachse, Wasser, organische Lösungsmittel, Parfüme, oberflächenaktive Mittel, öllösliche Vitamine, wasserlösliche Vitamine, öllösliche Arzneimittel, wasserlösliche Arzneimittel, UV-Absorber, aktive pharmazeutische Verbindungen und andere.

Insbesondere sind die erfindungsgemäßen Organopolysiloxangele in Antiperspirantien und Deoperspirantien geeignet, da sie ein trockenes Gefühl zurücklassen und die Haut nicht während der Verdampfung abkühlen. Sie sind gleitfähig und verbessern die Eigenschaften von Hautcremes, Hautpflegelotionen, Moisturizern, Gesichtsbehandlungen wie z.B. Akne- oder Faltenentfernern, Körper- und Gesichtsreinigern, Badeölen, Parfüms, Kölnisch Wasser, Sachets, Sonnenschutzmittel, Preshave- und Aftershave-Lotionen, flüssigen Seifen, Rasierseifen und Rasierschäumen. Sie können in Haarshampoos, Haarkonditionierern, Haarsprays, Mousses, Dauerwellenmittel, Haarentfernungsmittel und Nagelhautabdeckungen verwendet werden, um Glanz und Trockengleiten zu verbessern und Konditioniervorteile bereitzustellen.

In Kosmetika fungieren Sie als Verteilungsmittel für Pigmente in Make-ups, Farbkosmetika, Grundierungen, Rouge, Lippenstiften, Lippenbalsam, Lidstrich, Mascara, Ölentfernern und Farbkosmetikentfernern. Sie sind als Verabreichungssysteme für hierin beispielhaft genannte öllösliche aktive Bestandteile wie z.B. Vitamine, Arzneimittel und UV Absorber geeignet. Wenn sie in Stiften, Gelen, Lotionen, Roll-ons eingesetzt werden, verleihen die Elastomere ein trockenes, seidig sanftes Gefühl. Wenn in Kosmetika und anderen Hautpflegeprodukten eingebracht, verleihen die Elastomere einen Mattierungseffekt.

Zusätzlich zeigen die Organopolysiloxangele eine Vielzahl vorteilhafter Eigenschaften wie z.B. Klarheit, Lagerstabilität und Einfachheit der Herstellung. Daher haben sie einen breiten Anwendungsbereich, insbesondere in Antiperspirantien, Deodorantien, Hautpflegeprodukten, in Parfüms als Träger und für die Haarkonditionierung.

Die Organopolysiloxangele haben Verwendung über den Körperpflegebereich hinaus, einschließlich deren Verwendung als Füllstoff oder Isolierungsmaterial für elektrische Kabel, Boden- oder Wasserbarrieren für Bodenstabilisierung oder als Ersatz für Epoxymaterialen die in Bauteilen in der elektronischen Industrie verwendet werden. Sie sind ebenfalls als Träger für vernetzte Siliconkautschukteilchen geeignet. In diesen Anwendungen erlauben sie (i) die Einfachheit des Einbringens von Teilchen in solche Silicon- oder organische Phasen, wie Dichtmittel, Farben, Beschichtungen, Fetten, Klebstoffen, Antischaummitteln und Gießharzverbindungen, und (ii) stellen modifizierte rheologische, physikalische oder energieabsorbierende Eigenschaften solcher Phasen, entweder in ihrem reinen oder in ihrem Endzustand, bereit.

Zusätzlich sind die Organopolysiloxangele in der Lage, als Träger für Pharmazeutika, Biozide, Herbizide, Pestizide und andere biologische aktive Substanzen zu wirken.

Weiterhin finden die Zusammensetzungen Anwendung als Additive für nichtgewebte Trägersubstrate auf Cellulosebasis oder nichtgewebte synthetische Trägersubstrate, die in feuchten Reinigungstüchern wie Feuchttüchern, feuchten Papiertüchern und feuchten Handtüchern verwendet werden, die im Allgemeinen für Körperhygiene und Haushaltsreinigungszwecke vermarktet werden.

### Gelzubereitung, Allgemeine Vorschrift (A und B)

Nach Methode A wird zunächst ein "Basisgel" hergestellt, welches nach der Gelierung durch Zugabe einer weiteren Menge Verdünnungsmittel verdünnt wird. Methode B unterscheidet sich von Methode A grundsätzlich dadurch, dass von Beginn an die volle Menge Verdünnungsmittel zugegeben wird. Es findet keine nachträgliche Verdünnung des erhaltenen Gels statt.

### Vorschrift A:

Ein 2000 ml-Reaktionsgefäß aus Glas wird mit einem Kühler mit aufgesetzter Stickstoffeinleitung, Heizmantel, Ankerrührer und Temperaturregelung ausgestattet. Das Reaktionsgefäß wird vor Beginn der Reaktion 5 min mit Stickstoff gespült. Eine entsprechende Menge Verdünnungsmittel, der(die) Si-H-haltige(n) Vernetzer und das ungesättigte Organopolysiloxanharz werden zugegeben und bis zur vollständigen Lösung des Harzes gerührt. Der Hydrosilylierungskatalysator wird zugegeben und die Reaktionsmischung wird bei einer Rührgeschwindigkeit von etwa 200 U/min auf 95 °C erhitzt. Es wird 2,5 Stunden bei dieser Temperatur gerührt. Anschließend wird der Heizmantel entfernt und unter reduzierter Rührgeschwindigkeit (ca. 50 U/min) auf Raumtemperatur abgekühlt und Katalysatorgift zugesetzt. Das erhaltene Gel wird unter Schwenken eine Minute mit einem ULTRA-TURRAX® T 50 bei 6000 U/min homogenisiert. Man erhält ein "Basisgel", welches eine cremige bis feste oder bröckelige Konsistenz haben kann und für die Verwendung in Kosmetikprodukten geeignet ist.
Für die Verdünnung wird die gewünschte Menge Verdünnungsmittel zugegeben und bei 50 U/min mit dem Ankerrührer gerührt, bis das Verdünnungsmittel vollständig vom Gel aufgenommen worden ist (ca. 10 Minuten). Anschließend wird wie erneut unter Schwenken eine Minute mit einem ULTRA-TURRAX® T 50 bei 6000 U/min homogenisiert. Auf diesem Weg erhält man ein lagerstabiles, cremiges, transparentes oder transluzentes Gel mit sehr glatter Konsistenz, das zur Verwendung in Kosmetikprodukten geeignet ist.

### Vorschrift B:

Ein 2000 ml-Reaktionsgefäß aus Glas wird mit einem Kühler mit aufgesetzter Stickstoffeinleitung, Heizmantel, Ankerrührer und Temperaturregelung ausgestattet. Das Reaktionsgefäß wird vor Beginn der Reaktion 5 min mit Stickstoff gespült. Das Verdünnungsmittel, der Si-H-funktionelle Vernetzer und das ungesättigte Organopolysiloxanharz werden zugegeben und bis zur vollständigen Lösung des Harzes gerührt. Der Hydrosilylierungskatalysator wird zugegeben und die Reaktionsmischung wird bei einer Rührgeschwindigkeit von etwa 200 U/min auf 95 °C erhitzt. Es wird 2,5 Stunden bei dieser Temperatur gerührt. Anschließend wird der Heizmantel entfernt und unter reduzierter Rührgeschwindigkeit (ca. 50 U/min) auf Raumtemperatur abgekühlt und Katalysatorgift zugesetzt. Das erhaltene Gel wird unter Schwenken zwei Minuten mit einem ULTRA-TURRAX® T 50 bei 6000 U/min homogenisiert. Auf diesem Weg erhält man ein lager-stabiles, cremiges, transparentes oder transluzentes Gel mit sehr glatter Konsistenz, das zur Verwendung in Kosmetikprodukten geeignet ist.

### Beispiel 1 - 11 und Vergleichsbeispiel V1 - V5:

Nach den Vorschriften A und B wurde eine Reihe von Gelen hergestellt. Die verwendeten Substanzen, deren Mengen und die Eigenschaften der hergestellten Gele sind in den nachstehenden Tabellen 1 bis 3 wiedergegeben. Die Eigenschaften der in den Beispielen und Vergleichsbeispielen verwendeten Si-H-funktionellen Vernetzer sind in Tabelle 4 dargestellt.

Beispiele 1 - 5 sind Beispiele für Gele, bei denen ein Vernetzer mit einem sehr niedrigen Gehalt an Si-H Gruppen verwendet wird, als einziger Vernetzer oder in Kombination mit einem weiteren Vernetzer. Als Verdünnungsmittel wurde nichtflüchtiges Polydimethylsiloxan (5 mm²/s bei 25°C) gewählt. Es werden lagerstabile, cremige Gele erhalten, welche für die Verwendung in kosmetischen Formulierungen geeignet sind. Beispiel 3 zeigt ein hochviskoses "Basisgel". Die Gele aus den Beispielen 4 und 5 haben eine vergleichbare Zusammensetzung, wurden aber nach den unterschiedlichen Verfahren A bzw. B hergestellt. Die Gele zeigen sehr ähnliche Eigenschaften, Aussehen und Viskosität. Beispiel 4 wurde nach Vorschrift A aus dem "Basisgel" Beispiel 3 durch nachträgliche Verdünnung hergestellt. Beispiel 5 wurde dagegen nach Vorschrift B ohne Verdünnungsschritt hergestellt. Die Vergleichsbeispiele V1 und V2 zeigen Gele im gleichen Verdünnungsmittel wie in den Beispielen 1 - 5 verwendet. Vergleichsbeispiel V1 enthält jedoch einen Vernetzer mit sehr hohem Gehalt an Si-H-Gruppen, wie in US 6,423,322 B1 offenbart. Gel V1 hat eine für kosmetische Anwendungen unerwünschte ölige und fließende Konsistenz. Vergleichsbeispiel V2 enthält ausschließlich einen Vernetzer mit mittlerem Gehalt an Si-H Gruppen. Das Gel entmischt sich während der Lagerung in 2 Phasen. Es ist nicht lagerstabil und daher für kosmetische Anwendungen ungeeignet. Beispiel 6 zeigt ein Organopolysiloxangel, welches unter Verwendung von flüchtigen, linearen Polydimethylsiloxan (2,3 mm²/s bei 25°C) als Verdünnungsmittel hergestellt worden ist. Das Gel ist cremig, lagerstabil und transparent und für die Verwendung in kosmetischen Anwendungen sehr gut geeignet.

Beispiele 7 bis 9 zeigen Gele, bei denen ein Vernetzer mit einem sehr niedrigen Gehalt an Si-H Gruppen verwendet wird, als einziger Vernetzer oder in Kombination mit einem weiteren Vernetzer. Als Verdünnungsmittel wurde Isopropylmyristat (=Myristinsäurepropylester) gewählt. Es werden lagerstabile, cremige und transparente Gele erhalten, welche für die Verwendung in kosmetischen Formulierungen geeignet sind. Vergleichsbeispiele V3 und V4 zeigen Gele im gleichen Verdünnungsmittel, welche ausschließlich Vernetzer mit vergleichsweise hohem Gehalt an Si-H Gruppen beinhalten. Die Gele sind flüssig und nicht für kosmetische Anwendungen geeignet. V3 trennt sich zudem in zwei Phasen.

Beispiele 10 und 11 zeigen Gele, bei denen ein Vernetzer mit einem sehr niedrigen Gehalt an Si-H Gruppen verwendet wird, als einziger Vernetzer oder in Kombination mit einem weiteren Vernetzer. In diesen Beispielen wird Isododecan als Verdünnungsmittel. Es werden lagerstabile, cremige Gele erhalten, welche für die Verwendung in kosmetischen Formulierungen geeignet sind. Vergleichsbeispiel V5 zeigt ein Gel im gleichen Verdünnungsmittel, bei dem ein Vernetzer mit sehr hohem Gehalt an Si-H-Gruppen verwendet wird, wie in US 6,423,322 B1 offenbart. Es wird ein für kosmetische Anwendungen unerwünscht fließendes, öliges Gel erhalten, welches sich während der Lagerung in zwei Phasen entmischt.

Die Viskositäten der Organopolysiloxangele wurden nach DIN EN ISO 3219 bei Schergeschwindigkeit 1/s und 25°C bestimmt.

**TABELLE 1 - ELASTOMERGEL FORMULIERUNGEN:**

| Beispiel:¹ | | 1 | 2 | 3 | 4 | 5 | V1 | V2 |
|---|---|---|---|---|---|---|---|---|
| Verdünnungsmittel | Polydimethylsiloxan (5 mm²/s)² | 75, 67 | 79,76 | 75,65 | 82,75 | 82,65 | 79,73 | 79,47 |
| | Isopropyl-myristat³ | - | - | - | - | - | - | - |
| | Isododecan⁴ | - | - | - | - | - | - | - |
| | Polydimethylsiloxan (2,3 mm²/s) | - | - | - | - | - | - | - |
| ungesättigtes Silikonharz⁵ | | 16,15 | 10, 90 | 8,33 | 5,90 | 5,90 | 18,00 | 14,62 |
| Si-H-haltiger Vernetzer | Nr. 1 (0,46 % H) | - | - | - | - | - | 1,91 | - |
| | Nr.2 (0,14 % H) | 5,17 | 2,63 | - | - | - | - | 5,21 |
| | Nr. 3 (0,12 % H) | - | - | - | - | - | - | - |
| | Nr. 4 (0, 026 % H) | 2,59 | 6,37 | 15,59 | 11,04 | 11,03 | - | - |
| Platingift | Mercaptoöl⁶ | 0,41 | 0,35 | 0,43 | 0,30 | 0, 42 | 0,35 | 0,70 |
| Katalysator (ppm) | Platin-Komplex⁷ | 5 | 5 | 5 | 5 | 5 | 5 | 10 |
| Ansatzgröße (g) | | 559 | 1239 | 1501 | 2119 | 943 | 1003 | 1235 |
| Mol Harz-Vinyl / Mol Si-H | | 1,47 | 1,46 | 1,46 | 1,46 | 1,46 | 1,45 | 1,44 |
| Viskosität (mPa*s bei 25°C) | | 117000 | 127000 | 602000 | 160000 | 164000 | 84000 | 21000 |
| Eigenschaften | | cremig, standfest | cremig, standfest | klumpig, standfest | cremig, standfest | cremig, standfest | ölig bis pastös, leicht fließend | ölig, fließend, zwei-phasig |
| Aussehen | | transparent | transparent | transluzent | transparent | transparent | transluzent | transluzent |
| Lagerstabil | | ja | ja | ja | ja | ja | ja | nein |
| Gew.-% Elastomer im "Basisgel" (bei Methode A) | | - | 24 | - | 24 | - | - | 24 |
| Gew.-% Elastomer im fertigen Gel | | 24 | 20 | 24 | 17 | 17 | 20 | 20 |
| Verwendete Vorschrift | | B | A | B | A | B | B | A |

| | | | | | | | | |
|---|---|---|---|---|---|---|---|---|
| ¹Die Mengenangaben zu Verdünnungsmittel, ungesättigtes Silikonharz, Si-H funktioneller Vernetzer und Platingift sind zu verstehen als Gew.-% Anteil im fertigen Gel; ²WACKER-BELSIL® DM 5 zu beziehen bei Wacker Chemie AG; Viskosität bei 25°C; ³Isopropylmyristat (CAS: 110-27-0) zu beziehen bei Merck Schuchardt OHG; ⁴PUROLAN IDD (CAS: 93685-81-5) zu beziehen bei LANXESS Distribution GmbH; ⁵Verhältnis M / M^{Vi} / Q = 7,6 / 1 / 11,4, Mₙ = 2570, M_{w} = 5440, Jodzahl = 18; ⁶Polysiloxan mit 3-Mercaptopropylgruppen; Viskosität 190 mm²/s bei 25 °C, Mercaptangehalt 0,29 Gew.-%; ⁷WACKER® CATALYST OL zu beziehen bei Wacker Chemie AG; | | | | | | | | |

**TABELLE 2 - ELASTOMERGEL FORMULIERUNGEN:**

| Beispiel:¹ | | 6 | 7 | 8 | 9 | V3 | V4 |
|---|---|---|---|---|---|---|---|
| Verdünnungsmittel | Polydimethylsiloxan, (5 mm²/s)² | - | - | - | - | - | - |
| | Isopropyl-myristat³ | - | 75,37 | 75,68 | 75,65 | 75,16 | 75, 36 |
| | Isododecan⁴ | - | - | - | - | - | - |
| | Polydimethylsiloxan, (2,3 mm²/s) | 82,75 | - | - | - | - | - |
| ungesättigtes Silikonharz⁵ | | 5,90 | 13,48 | 12,73 | 8,33 | 16,97 | 17,54 |
| Si-H-haltiger Vernetzer | Nr. 1 (0,46 % H) | - | - | - | - | - | - |
| | Nr. 2 (0,14 % H) | - | 3,44 | - | - | - | 6,26 |
| | Nr. 3 (0,12 % H) | - | - | 3,72 | - | 7,22 | - |
| | Nr. 4 (0,026 % H) | 11, 04 | 6,88 | 7,44 | 15, 59 | - | - |
| Platingift | Mercaptoöl⁶ | 0,30 | 0,83 | 0,43 | 0,43 | 0,65 | 0,84 |
| Katalysator (ppm) | Platin-Komplex⁷ | 5 | 10 | 5 | 5 | 10 | 10 |
| Ansatzgröße (g) | | 706 | 520 | 1002 | 1001 | 532 | 515 |
| Mol Harz-Vinyl / mol Si-H | | 1,46 | 1,46 | 1,44 | 1,46 | 1, 44 | 1,44 |
| Viskosität (mPa*s bei 25°C) | | 129000 | 60000 | 56000 | 132000 | - | - |
| Eigenschaften | | cremig, standfest | cremig, standfest | cremig, standfest | cremig, standfest | flüssig, zweiphasig | flüssig |
| Aussehen | | transparent | transparent | transparent | transparent | transparent | transparent |
| Lagerstabil | | ja | ja | ja | ja | - | - |
| Gew.-% Elastomer im "Basisgel" (bei Methode A) | | 24 | - | - | - | - | - |
| Gew.-% Elastomer im fertigen Gel | | 17 | 24 | 24 | 24 | 24 | 24 |
| Verwendete Vorschrift | | A | B | B | B | B | B |

| | | | | | | | |
|---|---|---|---|---|---|---|---|
| ¹Die Mengenangaben zu Verdünnungsmittel, ungesättigtes Silikonharz, Si-H funktioneller Vernetzer und Platingift sind zu verstehen als Gew.-% Anteil im fertigen Gel; ²WACKER-BELSIL® DM 5 zu beziehen bei Wacker Chemie AG; Viskosität bei 25°C; ³Isopropylmyristat (CAS: 110-27-0) zu beziehen bei Merck Schuchardt OHG; ⁴PUROLAN IDD (CAS: 93685-81-5) zu beziehen bei LANXESS Distribution GmbH; ⁵Verhältnis M / M^{Vi} / Q = 7,6 / 1 / 11,4, Mₙ = 2570, M_{w} = 5440, Jodzahl = 18; ⁶Polysiloxan mit 3-Mercaptopropylgruppen; Viskosität 190 mm²/s bei 25 °C, Mercaptangehalt 0,29 Gew.-%; ⁷WACKER® CATALYST OL zu beziehen bei Wacker Chemie AG; | | | | | | | |

**TABELLE 3 - ELASTOMERGEL FORMULIERUNGEN:**

| Beispiel:¹ | | 10 | 11 | V5 |
|---|---|---|---|---|
| Verdünnungsmittel | Polydimethylsiloxan, (5 mm²/s)² | - | - | - |
| | Isopropylmyristat³ | - | - | - |
| | Isododecan⁴ | 75,36 | 82,57 | 79,73 |
| | Polydimethylsiloxan, (2, 3 mm²/s) | - | - | - |
| ungesättigtes Silikonharz⁵ | | 16,09 | 5,86 | 18,08 |
| Si-H-haltiger Vernetzer | Nr. 1 (0,46 % H) | - | - | 1, 92 |
| | Nr. 2 (0,14 % H) | 5,15 | - | - |
| | Nr. 3 (0,12 % H) | - | - | - |
| | Nr. 4 (0,026 % H) | 2,58 | 10,96 | - |
| Platingift | Mercaptoöl⁶ | 0,82 | 0,61 | 0,27 |
| Katalysator (ppm) | Platin-Komplex⁷ | 10 | 10 | 5 |
| Ansatzgröße (g) | | 561 | 711 | 416 |
| Mol Harz-Vinyl / Si-H | | 1,47 | 1,46 | 1,45 |
| Viskosität (mPa*s bei 25°C) | | 125000 | 117000 | 15000 |
| Eigenschaften | | cremig, standfest | cremig, standfest | ölig, fließend |
| Aussehen | | transparent | transparent | transparent |
| Lagerstabil | | ja | ja | nein |
| Gew.-% Elastomer im "Basisgel" (bei Methode A) | | - | 24 | 24 |
| Gew.-% Elastomer im fertigen Gel | | 24 | 17 | 20 |
| Verwendete Vorschrift | | B | A | A |

| | | | | |
|---|---|---|---|---|
| ¹Die Mengenangaben zu Verdünnungsmittel, ungesättigtes Silikonharz, Si-H funktioneller Vernetzer und Platingift sind zu verstehen als Gew.-% Anteil im fertigen Gel; ²WACKER-BELSIL® DM 5 zu beziehen bei Wacker Chemie AG; Viskosität bei 25°C; ³Isopropylmyristat (CAS: 110-27-0) zu beziehen bei Merck Schuchardt OHG; ⁴PUROLAN IDD (CAS: 93685-81-5) zu beziehen bei LANXESS Distribution GmbH; ⁵Verhältnis M / M^{Vi} / Q = 7,6 / 1 / 11,4, Mₙ = 2570, M_{w} = 5440, Jodzahl = 18; ⁶Polysiloxan mit 3-Mercaptopropylgruppen; Viskosität 190 mm²/s bei 25 °C, Mercaptangehalt 0,29 Gew.-%; ⁷WACKER® CATALYST OL zu beziehen bei Wacker Chemie AG; | | | | |

**TABELLE 4 - EIGENSCHAFTEN DER IN BEISPIEL 1 - 11 und Vergleichsbeispielen V1 - V5 VERWENDETEN SI-H-HALTIGEN VERNETZER:**

| **Nr.** | **Verteilung a: (b+c)** | **Summe a+b** | **Viskosität¹ (mm²/s bei 25°C)** | **% H** |
|---|---|---|---|---|
| | | | | |
| 1 | 2 : 1 | 138 | 331 | 0.47 |
| 2 | 9 : 1 | 60 | 58 | 0.14 |
| 3 | 11 : 1 | 130 | 328 | 0.12 |
| 4 | 55 : 1 | 134 | 321 | 0.026 |

| | | | | |
|---|---|---|---|---|
| ¹Gemessen bei 25 °C. | | | | |

### Beispiel 12:

Die Evaluierung der sensorischen Eigenschaft der Organopolysiloxangele aus Beispiel 1, Beispiel 2, Beispiel 4 und Vergleichsbeispiel V1 erfolgte durch eine geschulte Gruppe von Probanden. Hierfür wurden die Organopolysiloxangele, sofern notwendig, durch Zugabe von Polydimethylsiloxan (5 mm2/s) auf vergleichbare Viskosität (90000 mPa*s (+/-10%)) verdünnt. Dies gewährleistet, dass der sensorische Eindruck der Organopolysiloxangele nicht durch unterschiedliche Gel-Viskositäten verändert wird. Nach dem Auftragen auf die Haut wurden die sensorischen Eigenschaften der Rückstände relativ zueinander bewertet. Tabelle 5 zeigt die durchschnittliche Bewertung der Probanden, wobei die Note 5 einem bevorzugten samtig-seidenen Hautgefühl entspricht und die Note 0 einem unerwünschten fettig-öligen Hautgefühl entspricht.

**TABELLE 5 - BEWERTUNG DER SENSORISCHE EIGENSCHAFTEN:**

| **Organopolysiloxangel** | **Note** (Skala 0 bis 5; 5 = bestmögliche Bewertung) |
|---|---|
| Beispiel 1 | 5 |
| Beispiel 2 | 5 |
| Beispiel 4 | 4 |
| Vergleichsbeispiel V1 | 1 |

### Beispiel 13 - 14, Vergleichsbeispiel 6:

Eine transparente Fluid Foundation wurden mit den in Beispiel 2, Beispiel 4 und Vergleichsbeispiel V1 hergestellten Organopolysiloxangelen und den in Tabelle 6 gezeigten anderen Bestandteilen formuliert. Hierfür wurden die Organopolysiloxangele aus Beispiel 2 und 4 durch Zugabe von Verdünnungsmittel auf gleiche Viskosität (90000 mPa*s (+/-10%)) verdünnt, um bessere Vergleichbarkeit zu gewährleisten, wie in Beispiel 12 beschrieben. Die Evaluierung der sensorischen Eigenschaften erfolgte durch eine geschulte Gruppe von Probanden. Die Probanden bewerteten die Verteilbarkeit auf der Haut, sowie die Schlüpfrigkeit und Klebrigkeit des Rückstandes nach dem Verteilen. Tabelle 7 zeigt die durchschnittliche Bewertung der Probanden.

Zur Herstellung der Fluid Foundation werden zunächst die Öle der Phase A mittels Flügelrührer gemischt. Anschließend wird das Harz der Phase A unter Rühren und leichtem Erhitzen zugefügt bis eine einheitliche Mischung entsteht. Die Organopolysiloxangele werden zur Phase A gegeben und homogen eingerührt, wobei die Gele aus Beispiel 2 bzw. Beispiel 4 zuvor mit der angegebenen Menge WACKER-BELSIL® DM 5 verdünnt werden. Die Bestandteile der Phase B werden miteinander kombiniert und gemischt, bis sie einheitlich sind. Phase B wird zur Phase A gegeben und auf 75°C erhitzt. Die Bestandteile der Phase D werden unter Rühren homogen vermischt und auf 75°C erhitzt. Die Bestandteile der Phase C werden kombiniert und zur Phase D gegeben. Anschließend wird die Mischung CD homogenisiert. Die Mischung AB wird zur Mischung CD gegeben. Die Mischung wird homogenisiert und unter langsamen Kühlen auf Raumtemperatur gebracht, wobei bei einer Temperatur von 40°C die homogen verrührte Mischung der Bestandteile aus Phase E zugegeben werden.

Es zeigt sich äußerst überraschend, dass die erfindungsgemäßen Organopolysiloxangele aus Beispiel 2 und Beispiel 4, welche zuvor mit der angegebenen Menge Verdünnungsmittel auf vergleichbare Viskosität wie Vergleichsbeispiel V1 verdünnt worden sind, als Bestandteil einer Fluid Foundation nicht nur eine wesentliche Verbesserung der sensorischen Eigenschaften bewirkten, sondern dass hierfür auch eine geringere Menge an Silicon Elastomer ausreichend ist. Dies ergibt sich aus dem Mengenanteil in der Foundation (Tabelle 6) unter Berücksichtigung des Gew.-% Gehalts an Elastomer (Tabelle 1).

**TABELLE 6 - TRANSPARENTE FLUID FOUNDATION:**

| **Phase** | **Handelsname** | **INCI-Name** | **Vergleichsbeispiel 6** | **Beispiel 13** | **Beispiel 14** |
|---|---|---|---|---|---|
| A | Wacker-Belsil® TMS 803 | Trimethylsiloxysilicate | 1,9 | 1, 9 | 1,9 |
| A | Crodamol PMP | PPG-2 Myristyl Ether Propionate | 0,8 | 0, 8 | 0,8 |
| A | Eusolex® OS | Ethylhexyl Salicylate | 4,8 | 4, 8 | 4,8 |
| A | Miglyol® 812 N | Caprylic/Capric Triglyceride | 1,9 | 1, 9 | 1,9 |
| A | Octyldodecyl Neopentanoate | Octyldodecyl Neopentanoate | 1 | 1 | 1 |
| | Vergleichsbeis piel V1 | Dimethicone, Dimethicone/ Vinyltrimethyl Siloxysilicate Crosspolymer | 7,7 | | |
| | Beispiel 2 | Dimethicone, Dimethicone/ Vinyltrimethyl Siloxysilicate Crosspolymer | | 7, 15 | |
| | Beispiel 4 | Dimethicone, Dimethicone/ Vinyltrimethyl Siloxysilicate Crosspolymer | | | 6,96 |
| | Wacker-Belsil® DM 5 | Dimethicone | | 0, 55 | 0,74 |
| B | Wacker-Belsil® CDM 3526 VP | C26-28 Alkyl Dimethicone | 1,9 | 1, 9 | 1,9 |
| B | Eusolex® 9020 | Butyl Methoxydibenzoyl methane | 1,9 | 1, 9 | 1,9 |
| B | Span 85 | Sorbitan Trioleate | 1,9 | 1, 9 | 1,9 |
| B | Tegin® | Glyceryl Stearate SE | 1,9 | 1, 9 | 1,9 |

| **Phase** | **Handelsname** | **INCI-Name** | **Vergleichsbeispiel V6** | **Beispiel 13** | **Beispiel 14** |
|---|---|---|---|---|---|
| C | AEC Magnesium Aluminium Silicate G2 | Magnesium Aluminum Silicate | 0,46 | 0,46 | 0,46 |
| C | Covafluid AMD | Aluminum Starch Octenylsuccinate | 0,9 | 0,9 | 0,9 |
| C | Eusolex® T 2000 | Titanium Dioxide, Alumina, Simethicone | 2,9 | 2,9 | 2,9 |
| C | Luzenac Pharma UM | Talc | 1,9 | 1,9 | 1,9 |
| C | Tres BN® PUHP1109 | Boron Nitride | 0,1 | 0,1 | 0,1 |
| C | Pigment Pre Mix¹ | | 4,74 | 4,74 | 4,74 |
| D | Butylenglykol | Butylene Glycol | 2,9 | 2,9 | 2,9 |
| D | EDETA B Pulver | Tetrasodium EDTA | 0,3 | 0,3 | 0,3 |
| D | Glycerin 100% waterless | Glycerin | 2,9 | 2,9 | 2,9 |
| D | Keltrol SF | Xanthan Gum | 0,5 | 0,5 | 0,5 |
| D | Tween 60 | Polysorbate 60 | 1,9 | 1,9 | 1,9 |
| D | Water | Aqua (DI Water) | 53,75 | 53,75 | 53,75 |
| E | Copherol 1250 | Tocopheryl Acetate | 0,3 | 0,3 | 0,3 |
| E | Parfum SCE 243993 Pitanga | Parfum | 0,25 | 0,25 | 0,25 |
| E | Phenonip | Phenoxyethanol and Methylparaben and Ethylparaben and Butylparaben and Propylparaben and Isobutylparaben | 0,5 | 0,5 | 0,5 |

| | | | | | |
|---|---|---|---|---|---|
| ¹3,95 Teile CI 77891 Unipure White LC 981 (LCW), 2,90 Teile Titanium Dioxide, Alumina, Simethicone Eusolex T 2000 (Merck KGaA), 0,15 Teile CI 77491 Unipure Red LC 383 (LCW), 0,10 Teile Boron Nitride Tres BN PUHP1109 (Saint-Gobain Advanced Ceramics Bor), 0,04 Teile CI 77499 Unipure Black LC 989 (LCW), 0,50 Teile CI 77492 Unipure Yellow LC 181 (LCW), 0,10 Teile CI 77491+CI 77492+CI 77499 Unipure Brown LC 887 (LCW). | | | | | |

**TABELLE 7 - BEWERTUNG DER SENSORISCHE EIGENSCHAFTEN:**

| **Fluid Foundation** | **Verteilung Foundation** | **Schlüpfrigkeit Rückstand** | **Klebrigkeit Rückstand** |
|---|---|---|---|
| Vergleichsbeispiel V6 (beinhaltend V1) | ○ | ○ | ○ |
| Beispiel 13 (beinhaltend Beispiel 2) | ++ | + | ○ |
| Beispiel 14 (beinhaltend Beispiel 4) | ○ | + | ++ |

## Patentansprüche

1. Organopolysiloxangele hergestellt durch Umsetzung von
(1) ungesättigten MQ-Harze aus Einheiten der Formeln
SiO₂ (Q-Einheiten) und
R₃SiO_{1/2} und R₂R'SiO_{1/2} (M-Einheiten)
wobei R gleich oder verschieden sein kann und einen einwertigen, gegebenenfalls substituierten Kohlenwasserstoffrest mit 1 bis 18 Kohlenstoffatomen je Rest bedeutet,
R' einen einwertigen Kohlenwasserstoffrest bedeutet, an den Si-H-Gruppen in einer Hydrosilylierungsreaktion angelagert werden können, vorzugsweise ein
ω-Alkenylrest mit 2 bis 12 C-Atomen, bevorzugt ein Vinylrest ist,
mit der Maßgabe, dass die MQ-Harze mindestens 2 Reste R', vorzugsweise mindestens 3 Reste R', enthalten und dass das molare Verhältnis von M-Einheiten zu Q-Einheiten im Bereich von 0,5 bis 4,0, vorzugsweise im Bereich von 0,5 bis 2,0, bevorzugt im Bereich von 0,6 bis 1,5, liegt,
mit
(2) Si-H funktionellen Organopolysiloxanen der allgemeinen Formel
H_{c}R_{3-c}SiO(R₂SiO)ₐ(RHSiO)_{b}SiR_{3-c}H_{c} (I),
wobei
c 0 oder 1, vorzugsweise 0, ist,
R gleich oder verschieden sein kann und einen einwertigen, gegebenenfalls substituierten Kohlenwasserstoffrest mit 1 bis 18 Kohlenstoffatomen je Rest bedeutet,
a und b ganze Zahlen sind, mit der Maßgabe, dass die Summe a+b 66 bis 248, vorzugsweise 98 bis 248, bevorzugt 118 bis 168 ist,
dass die Organopolysiloxane (2) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten,
und dass die Anzahl an Si-H-Gruppen pro Molekül durchschnittlich größer 2 ist,
oder Mischungen von (2) Si-H funktionellen Organopolysiloxanen mit
(2') Si-H funktionellen Organopolysiloxanen der allgemeinen Formel
H_{c}R_{3-c}SiO(R₂SiO)ₐ(RHSiO)_{b}SiR_{3-c}H_{c} (I'),
wobei
c 0 oder 1, vorzugsweise 0 ist,
R die oben dafür angegebene Bedeutung hat,
a und b ganze Zahlen sind, mit der Maßgabe, dass die Summe a+b 8 bis 248, vorzugsweise 38 bis 248, ist, und dass die Organopolysiloxane (2') Si-gebundenen Wasserstoff in Mengen von 0,045 bis 0,35 Gew.-%, vorzugsweise von 0,045 bis 0,156 Gew.-%, enthalten, mit der Maßgabe, dass wenn Mischungen von (2) und (2') eingesetzt werden, das Gewichts-Verhältnis von (2) zu (2') vorzugsweise größer 0,2, bevorzugt größer 0,3 ist,
in Gegenwart von
(3) die Anlagerung von Si-gebundenem Wasserstoff an aliphatische Mehrfachbindung fördernde Katalysatoren, wobei (1) und (2) oder Mischungen von (2) und (2') in
(4) Verdünnungsmitteln ausgewählt aus Organopolysiloxanen mit 2 bis 200 Si-Atomen und organischen Verdünnungsmitteln,
dispergiert sind.

2. Organopolysiloxangele nach Anspruch 1, **dadurch gekennzeichnet, dass** nach der Umsetzung durch anschließendes Homogenisieren cremige, lagerstabile Organopolysiloxangele erhalten werden.

3. Organopolysiloxangele nach Anspruch 2, **dadurch gekennzeichnet, dass** die so erhaltenen Organopolysiloxangele durch Zugabe von weiteren Verdünnungsmitteln (4) und/oder aktiven Wirkstoffen für die Körperpflege oder Gesundheitspflege und ggf. anschließendem Homogenisieren weiter verdünnt werden.

4. Organopolysiloxangele nach einem der Ansprüche 1 bis 3, **dadurch gekennzeichnet, dass** als Verdünnungsmittel (4) Polydimethylsiloxane mit 2 bis 50 Si-Atomen, aliphatische oder alicyclische Kohlenwasserstoffe mit 4 bis 30 C-Atomen oder Ester von Carbonsäuren mit 2 bis 30 C-Atomen eingesetzt werden.

5. Verfahren zur Herstellung der Organopolysiloxangele nach Anspruch 1, **dadurch gekennzeichnet, dass**
(1) ungesättigte MQ-Harze aus Einheiten der Formeln
SiO₂ (Q-Einheiten) und
R₃SiO_{1/2} und R₂R'SiO_{1/2} (M-Einheiten)
wobei R gleich oder verschieden sein kann und einen einwertigen, gegebenenfalls substituierten Kohlenwasserstoffrest mit 1 bis 18 Kohlenstoffatomen je Rest bedeutet,
R' einen einwertigen Kohlenwasserstoffrest bedeutet, an den Si-H-Gruppen in einer Hydrosilylierungsreaktion angelagert werden können, vorzugsweise ein
ω-Alkenylrest mit 2 bis 12 C-Atomen, bevorzugt ein Vinylrest ist,
mit der Maßgabe, dass die MQ-Harze mindestens 2 Reste R', vorzugsweise mindestens 3 Reste R', enthalten und dass das molare Verhältnis von M-Einheiten zu Q-Einheiten im Bereich von 0,5 bis 4,0, vorzugsweise im Bereich von 0,5 bis 2,0, bevorzugt im Bereich von 0,6 bis 1,5, liegt,
mit
(2) Si-H funktionellen Organopolysiloxanen der allgemeinen Formel
H_{c}R_{3-c}SiO(R₂SiO)ₐ(RHSiO)_{b}SiR_{3-c}H_{c} (I),
wobei
c 0 oder 1, vorzugsweise 0, ist,
R gleich oder verschieden sein kann und einen einwertigen, gegebenenfalls substituierten Kohlenwasserstoffrest mit 1 bis 18 Kohlenstoffatomen je Rest bedeutet,
a und b ganze Zahlen sind, mit der Maßgabe, dass die Summe a+b 66 bis 248, vorzugsweise 98 bis 248, bevorzugt 118 bis 168 ist,
dass die Organopolysiloxane (2) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten,
und dass die Anzahl an Si-H-Gruppen pro Molekül durchschnittlich größer 2 ist,
oder Mischungen von (2) Si-H funktionellen
Organopolysiloxanen mit
(2') Si-H funktionellen Organopolysiloxanen der allgemeinen Formel
H_{c}R_{3-c}SiO(R₂SiO)ₐ(RHSiO)_{b}SiR_{3-c}H_{c} (I'),
wobei
c 0 oder 1, vorzugsweise 0 ist,
R die oben dafür angegebene Bedeutung hat,
a und b ganze Zahlen sind, mit der Maßgabe, dass die Summe a+b 8 bis 248, vorzugsweise 38 bis 248, ist, und dass die Organopolysiloxane (2') Si-gebundenen Wasserstoff in Mengen von 0,045 bis 0,35 Gew.-%, vorzugsweise von 0,045 bis 0,156 Gew.-%, enthalten, mit der Maßgabe, dass wenn Mischungen von (2) und (2') eingesetzt werden, das Gewichts-Verhältnis von (2) zu (2') vorzugsweise größer 0,2, bevorzugt größer 0,3 ist,
in Gegenwart von
(3) die Anlagerung von Si-gebundenem Wasserstoff an aliphatische Mehrfachbindung fördernde Katalysatoren, umgesetzt werden,
wobei (1) und (2) oder Mischungen von (2) und (2') in
(4) Verdünnungsmitteln ausgewählt aus Organopolysiloxanen mit 2 bis 200 Si-Atomen und organischen Verdünnungsmitteln,
dispergiert sind.

6. Verfahren nach Anspruch 5, **dadurch gekennzeichnet, dass** die nach der Umsetzung erhaltenen Organopolysiloxangele homogenisiert werden, wobei cremige, lagerstabile Organopolysiloxangele erhalten werden.

7. Verfahren nach Anspruch 6, **dadurch gekennzeichnet, dass** die so erhaltenen Organopolysiloxangele mit weiteren Verdünnungsmittel (4) und/oder aktiven Wirkstoffen für die Körperpflege oder Gesundheitspflege verdünnt werden und ggf. anschließend homogenisiert werden.

8. Verfahren nach einem der Ansprüche 5 bis 7, **dadurch gekennzeichnet, dass** als Verdünnungsmittel (4) Polydimethylsiloxane mit 2 bis 50 Si-Atomen, aliphatische oder alicyclische Kohlenwasserstoffe mit 4 bis 30 C-Atomen oder Ester von Carbonsäuren mit 2 bis 30 C-Atomen eingesetzt werden.

9. Kosmetische Zusammensetzungen enthaltend Organopolysiloxangele nach einem der Ansprüche 1 bis 4 oder hergestellt nach einem der Ansprüche 5 bis 8.

## Claims

1. Organopolysiloxane gels produced by reaction of
(1) unsaturated MQ resins from units of the formulas
SiO₂ (Q units) and
R₃SiO_{1/2} and R₂R'SiO_{1/2} (M units),
wherein R can be identical or different and denotes a monovalent, optionally substituted hydrocarbon residue with 1 to 18 carbon atoms per residue,
R' denotes a monovalent hydrocarbon residue, onto which Si-H groups can be added in a hydrosilylation reaction, is preferably an
ω-alkenyl residue with 2 to 12 carbon atoms, preferably a vinyl residue,
with the proviso that the MQ resins contain at least 2 residues R', preferably at least 3 residues R', and that the molar ratio of M units to Q units is in the range from 0.5 to 4.0, preferably in the range from 0.5 to 2.0, preferably in the range from 0.6 to 1.5,
with
(2) Si-H functional organopolysiloxanes of the general formula
H_{c}R_{3-c}SiO(R₂SiO)ₐ(RHSiO)_{b}SiR_{3-c}H_{c} (I),
wherein
c is 0 or 1, preferably 0,
R can be identical or different and denotes a monovalent, optionally substituted hydrocarbon residue with 1 to 18 carbon atoms per residue,
a and b are integers, with the proviso that the sum a+b is 66 to 248, preferably 98 to 248, preferably 118 to 168,
that the organopolysiloxanes (2) contain Si-bonded hydrogen in amounts from 0.011 to 0.044 wt.%, preferably from 0.019 to 0.044 wt.%, preferably from 0.022 to 0.032 wt.%,
and that the number of Si-H groups per molecule on average is greater than 2,
or mixtures of (2) Si-H functional
organopolysiloxanes with
(2')Si-H functional organopolysiloxanes of the general formula
H_{c}R_{3-c}SiO(R₂SiO)ₐ(RHSiO)_{b}SᵢR_{3-c}H_{c} (I'),
wherein
c is 0 or 1, preferably 0,
R has the meaning given for it above,
a and b are integers, with the proviso that the sum a+b is 8 to 248, preferably 38 to 248, and that the organopolysiloxanes (2') contain Si-bonded hydrogen in amounts from 0.045 to 0.35 wt.%, preferably from 0.045 to 0.156 wt.%, with the proviso that if mixtures of (2) and (2') are used, the weight ratio of (2) to (2') is preferably greater than 0.2, more preferably greater than 0.3,
in the presence of
(3) the addition product of Si-bonded hydrogen onto catalysts promoting aliphatic multiple bonds,
wherein (1) and (2) or mixtures of (2) and (2') are dispersed in
(4) diluents, selected from organopolysiloxanes with 2 to 200 Si atoms and organic diluents.

2. Organopolysiloxane gels according to Claim 1, **characterized in that** after the reaction, creamy organopolysiloxane gels that are stable in storage are obtained by subsequent homogenization.

3. Organopolysiloxane gels according to Claim 2, **characterized in that** the organopolysiloxane gels thus obtained are further diluted by adding further diluents (4) and/or active substances for body care or health care and optionally undergo subsequent homogenization.

4. Organopolysiloxane gels according to any one of Claims 1 to 3, **characterized in that** polydimethylsiloxanes with 2 to 50 Si atoms, aliphatic or alicyclic hydrocarbons with 4 to 30 carbon atoms or esters of carboxylic acids with 2 to 30 carbon atoms are used as diluent (4).

5. Method of production of the organopolysiloxane gels according to Claim 1, **characterized in that**
(1) unsaturated MQ resins from units of the formulas
SiO₂ (Q units) and
R₃SiO_{1/2} and R₂R'SiO_{1/2} (M units),
wherein R can be identical or different and denotes a monovalent, optionally substituted hydrocarbon residue with 1 to 18 carbon atoms per residue,
R' denotes a monovalent hydrocarbon residue, onto which Si-H groups can be added in a hydrosilylation reaction, is preferably an
ω-alkenyl residue with 2 to 12 carbon atoms, preferably a vinyl residue,
with the proviso that the MQ resins contain at least 2 residues R', preferably at least 3 residues R', and that the molar ratio of M units to Q units is in the range from 0.5 to 4.0, preferably in the range from 0.5 to 2.0, preferably in the range from 0.6 to 1.5,
are reacted with
(2) Si-H functional organopolysiloxanes of the general formula
H_{c}R_{3-c}SiO(R₂SiO)ₐ(RHSiO)_{b}SiR_{3-c}H_{c} (I),
wherein
c is 0 or 1, preferably 0,
R can be identical or different and denotes a monovalent, optionally substituted hydrocarbon residue with 1 to 18 carbon atoms per residue,
a and b are integers, with the proviso that the sum a+b is 66 to 248, preferably 98 to 248, preferably 118 to 168,
that the organopolysiloxanes (2) contain Si-bonded hydrogen in amounts from 0.011 to 0.044 wt.%, preferably from 0.019 to 0.044 wt.%, preferably from 0.022 to 0.032 wt.%,
and that the number of Si-H groups per molecule on average is greater than 2,
or mixtures of (2) Si-H functional organopolysiloxanes with
(2')Si-H functional organopolysiloxanes of the general formula
H_{c}R_{3-c}SiO(R₂SiO)ₐ(RHSiO)_{b}SiR_{3-c}H_{c} (I'),
wherein
c is 0 or 1, preferably 0,
R has the meaning given for it above,
a and b are integers, with the proviso that the sum a+b is 8 to 248, preferably 38 to 248, and that the organopolysiloxanes (2') contain Si-bonded hydrogen in amounts from 0.045 to 0.35 wt.%, preferably from 0.045 to 0.156 wt.%, with the proviso that if mixtures of (2) and (2') are used, the weight ratio of (2) to (2') is preferably greater than 0.2, more preferably greater than 0.3,
in the presence of
(3) the addition product of Si-bonded hydrogen onto catalysts promoting aliphatic multiple bonds,
wherein (1) and (2) or mixtures of (2) and (2') are dispersed in
(4) diluents, selected from organopolysiloxanes with 2 to 200 Si atoms and organic diluents.

6. Method according to Claim 5, **characterized in that** the organopolysiloxane gels obtained after the reaction are homogenized, wherein creamy organopolysiloxane gels that are stable in storage are obtained.

7. Method according to Claim 6, **characterized in that** the organopolysiloxane gels thus obtained are diluted with further diluent (4) and/or active substances for body care or health care and optionally are subsequently homogenized.

8. Method according to any one of Claims 5 to 7, **characterized in that** polydimethylsiloxanes with 2 to 50 Si atoms, aliphatic or alicyclic hydrocarbons with 4 to 30 carbon atoms or esters of carboxylic acids with 2 to 30 carbon atoms are used as diluent (4).

9. Cosmetic compositions containing organopolysiloxane gels according to any one of Claims 1 to 4 or produced according to any one of Claims 5 to 8.

## Revendications

1. Gels d'organopolysiloxanes, produits par mise en réaction de
(1) résines MQ insaturées à base de motifs de formules SiO₂ (motifs Q) et
R₃SiO_{1/2} et R₂R'SiO_{1/2} (motifs M)
où R peut être le même ou différent et représente un radical hydrocarboné monovalent, éventuellement substitué, ayant de 1 à 18 atomes de carbone par radical,
R' représente un radical hydrocarboné monovalent, auquel peuvent être fixés par addition des groupes Si-H dans une réaction d'hydrosilylation, de préférence est un radical ω-alcényle ayant de 2 à 12 atomes de carbone, de façon particulièrement préférée un radical vinyle,
étant entendu que les résines MQ contiennent au moins 2 radicaux R', de préférence au moins 3 radicaux R', et que le rapport molaire des motifs M aux motifs Q se situe dans la plage de 0,5 à 4,0, de préférence dans la plage de 0,5 à 2,0, de façon particulièrement préférée dans la plage de 0,6 à 1,5,
avec
(2) des organopolysiloxanes à fonction Si-H de formule générale
H_{c}R_{3-c}SiO(R₂SiO)ₐ(RHSiO)_{b}SiR_{3-c}H_{c} (I),
dans laquelle
c est 0 ou 1, de préférence 0,
R peut être le même ou différent et représente un radical hydrocarboné monovalent, éventuellement substitué, ayant de 1 à 18 atomes de carbone par radical,
a et b sont des nombres entiers, étant entendu que la somme a+b vaut de 66 à 248, de préférence de 98 à 248, de façon particulièrement préférée de 118 à 168,
que les organopolysiloxanes (2) contiennent des atomes d'hydrogène liés à Si en des quantités de 0,011 à 0,044 % en poids, de préférence de 0,019 à 0,044 % en poids, de façon particulièrement préférée de 0,022 à 0,032 % en poids,
et que le nombre de groupes Si-H par molécule est en moyenne supérieur à 2,
ou des mélanges de (2) organopolysiloxanes à fonction
Si-H avec
(2') des organopolysiloxanes à fonction Si-H de formule générale
H_{c}R_{3-c}SiO(R₂SiO)ₐ(RHSiO)_{b}SiR_{3-c}H_{c} (I'),
dans laquelle
c est 0 ou 1, de préférence 0,
R a la signification indiquée ci-dessus pour ce radical,
a et b sont des nombres entiers, étant entendu que la somme a+b vaut de 8 à 248, de préférence de 38 à 248,
et que les organopolysiloxanes (2') contiennent des atomes d'hydrogène liés à Si en des quantités de 0,045 à 0,35 % en poids, de préférence de 0,045 à 0,156 % en poids,
étant entendu que lorsqu'on utilise des mélanges de (2) et (2'), le rapport pondéral de (2) à (2') est de préférence supérieur à 0,2, de façon particulièrement préférée supérieur à 0,3,
en présence de
(3) catalyseurs favorisant la fixation par addition d'hydrogène lié à Si sur une liaison multiple aliphatique,
(1) et (2) ou les mélanges de (2) et (2') étant dispersés dans
(4) des diluants choisis parmi des organopolysiloxanes ayant de 2 à 200 atomes de Si et des diluants organiques.

2. Gels d'organopolysiloxanes selon la revendication 1, **caractérisés en ce qu'**après la réaction on obtient par homogénéisation subséquente des gels d'organopolysiloxanes crémeux, stables au stockage.

3. Gels d'organopolysiloxanes selon la revendication 2, **caractérisés en ce qu'**on dilue davantage les gels d'organopolysiloxanes ainsi obtenus, par addition d'autres diluants (4) et/ou de substances actives pour le soin du corps ou les soins de santé et éventuellement homogénéisation subséquente.

4. Gels d'organopolysiloxanes selon l'une quelconque des revendications 1 à 3, **caractérisés en ce qu'**on utilise comme diluants (4) des polydiméthylsiloxanes ayant de 2 à 50 atomes de Si, des hydrocarbures aliphatiques ou alicycliques ayant de 4 à 30 atomes de carbone ou des esters d'acides carboxyliques ayant de 2 à 30 atomes de carbone.

5. Procédé pour la préparation des gels d'organopolysiloxanes selon la revendication 1, **caractérisé en ce qu'**on fait réagir
(1) des résines MQ insaturées à base de motifs de formules
SiO₂ (motifs Q) et
R₃SiO_{1/2} et R₂R'SiO_{1/2} (motifs M)
où R peut être le même ou différent et représente un radical hydrocarboné monovalent, éventuellement substitué, ayant de 1 à 18 atomes de carbone par radical,
R' représente un radical hydrocarboné monovalent, auquel peuvent être fixés par addition des groupes Si-H dans une réaction d'hydrosilylation, de préférence est un radical ω-alcényle ayant de 2 à 12 atomes de carbone, de façon particulièrement préférée un radical vinyle,
étant entendu que les résines MQ contiennent au moins 2 radicaux R', de préférence au moins 3 radicaux R', et que le rapport molaire des motifs M aux motifs Q se situe dans la plage de 0,5 à 4,0, de préférence dans la plage de 0,5 à 2,0, encore mieux dans la plage de 0,6 à 1,5,
avec
(2) des organopolysiloxanes à fonction Si-H de formule générale
H_{c}R_{3-c}SiO(R₂SiO)ₐ(RHSiO)_{b}SiR_{3-c}H_{c} (I),
dans laquelle
c est 0 ou 1, de préférence 0,
R peut être le même ou différent et représente un radical hydrocarboné monovalent, éventuellement substitué, ayant de 1 à 18 atomes de carbone par radical,
a et b sont des nombres entiers, étant entendu que la somme a+b vaut de 66 à 248, de préférence de 98 à 248, de façon particulièrement préférée de 118 à 168,
que les organopolysiloxanes (2) contiennent des atomes d'hydrogène liés à Si en des quantités de 0,011 à 0,044 % en poids, de préférence de 0,019 à 0,044 % en poids, de façon particulièrement préférée de 0,022 à 0,032 % en poids,
et que le nombre de groupes Si-H par molécule est en moyenne supérieur à 2,
ou des mélanges de (2) organopolysiloxanes à fonction Si-H avec
(2') des organopolysiloxanes à fonction Si-H de formule générale
H_{c}R_{3-c}SiO(R₂SiO)ₐ(RHSiO)_{b}SiR_{3-c}H_{c} (I'),
dans laquelle
c est 0 ou 1, de préférence 0,
R a la signification indiquée ci-dessus pour ce radical,
a et b sont des nombres entiers, étant entendu que la somme a+b vaut de 8 à 248, de préférence de 38 à 248,
et que les organopolysiloxanes (2') contiennent des atomes d'hydrogène liés à Si en des quantités de 0,045 à 0,35 % en poids, de préférence de 0,045 à 0,156 % en poids,
étant entendu que lorsqu'on utilise des mélanges de (2) et (2'), le rapport pondéral de (2) à (2') est de préférence supérieur à 0,2, de façon particulièrement préférée supérieur à 0,3,
en présence de
(3) catalyseurs favorisant la fixation par addition d'hydrogène lié à Si sur une liaison multiple aliphatique,
(1) et (2) ou les mélanges de (2) et (2') étant dispersés dans
(4) des diluants choisis parmi des organopolysiloxanes ayant de 2 à 200 atomes de Si et des diluants organiques.

6. Procédé selon la revendication 5, **caractérisé en ce qu'**on homogénéise les gels d'organopolysiloxanes obtenus après la réaction, pour obtenir des gels d'organopolysiloxanes crémeux, stables au stockage.

7. Procédé selon la revendication 6, **caractérisé en ce qu'**on dilue les gels d'organopolysiloxanes ainsi obtenus, avec d'autres diluants (4) et/ou des substances actives pour le soin du corps ou les soins de santé et éventuellement ensuite on les homogénéise.

8. Procédé selon l'une quelconque des revendications 5 à 7, **caractérisé en ce qu'**on utilise comme diluants (4) des polydiméthylsiloxanes ayant de 2 à 50 atomes de Si, des hydrocarbures aliphatiques ou alicycliques ayant de 4 à 30 atomes de carbone ou des esters d'acides carboxyliques ayant de 2 à 30 atomes de carbone.

9. Compositions cosmétiques contenant des gels d'organopolysiloxanes selon l'une quelconque des revendications 1 à 4 ou préparés selon l'une quelconque des revendications 5 à 8.
